Europäisches Patentamt

European Patent Office

Office européen des brevets

(19)

(11) Veröffentlichungsnummer: **0 137 375**
**B1**

(12) **EUROPÄISCHE PATENTSCHRIFT**

(45) Veröffentlichungstag der Patentschrift: **08.08.90**

(21) Anmeldenummer: **84111231.1**

(22) Anmeldetag: **20.09.84**

(51) Int. Cl.⁵: **C 07 D 401/06**, A 61 K 31/47

(54) **Mefloquin-Hydrochlorid.**

(30) Priorität: **07.10.83 CH 5459/83**

(43) Veröffentlichungstag der Anmeldung:
**17.04.85 Patentblatt 85/16**

(45) Bekanntmachung des Hinweises auf die
Patenterteilung:
**08.08.90 Patentblatt 90/32**

(84) Benannte Vertragsstaaten:
**AT BE CH DE FR GB IT LI NL**

(56) Entgegenhaltungen:
**EP-A-0 026 894**
**EP-A-0 092 185**
**FR-A-2 390 958**

(73) Patentinhaber: **F. HOFFMANN-LA ROCHE AG**
**Postfach 3255**
**CH-4002 Basel (CH)**

(72) Erfinder: **Bömches, Helmut**
**Käppelirainweg 26**
**CH-4147 Aesch (CH)**
Erfinder: **Hardegger, Bruno, Dr.**
**Bäumliweg 36**
**CH-4125 Riehen (CH)**

(74) Vertreter: **Lederer, Franz, Dr. et al**
**Van der Werth, Lederer & Riederer**
**Patentanwälte Lucile-Grahn-Strasse 22**
**D-8000 München 80 (DE)**

**Die Akte enthält technische Angaben, die nach
dem Eingang der Anmeldung eingereicht
wurden und die nicht in dieser Patentschrift
enthalten sind.**

Courier Press, Leamington Spa, England.

EP 0 137 375 B1

**Beschreibung**

Mefloquin-hydrochlorid erythro-α-2-Piperidyl-2,8-bis-(trifluormethyl)-4-chinolinmethanol-hydrochlorid, ist ein wertvoller Wirkstoff zur Behandlung der chloroquinresistenten Form der Malaria. Beidenjenigen Verfahren zur Herstellung von Mefloquin, deren letzte Stufe in der katalytischen Hydrierung des 2-Pyridyl-2,8-bis-(trifluormethyl)-4-chinolylketons besteht, fällt neben der biologisch aktiven erythro-Form auch stets eine geringere Menge (ca. 5—15%) der inaktiven und daher unerwünschten threo-Form an.

Die Abtrennung der threo- von der erythro-Form und die Reindarstellung der letzteren kann dadurch erreicht werden, dass man in einer ersten Stufe das bei der Hydrierung anfallende Gemisch von erythro- und threo-α-2-Piperidyl-2,8-bis-(trifluormethyl)-4-chinolinmethanol-hydrochlorid mit wässrigem Methanol oder Aethanol (ca. 60 bis 95 Vol% Wasser) behandelt, wobei die threo-Form in Lösung geht und die erythro-Form zurückbleibt. In einer zweiten Stufe können Reste der threo-Verbindung sowie anderer Nebenprodukte durch weitere Reinigung, beispielsweise durch Behandlung mit Aceton (d.h. via Aceton-Komplex), entfernt werden.

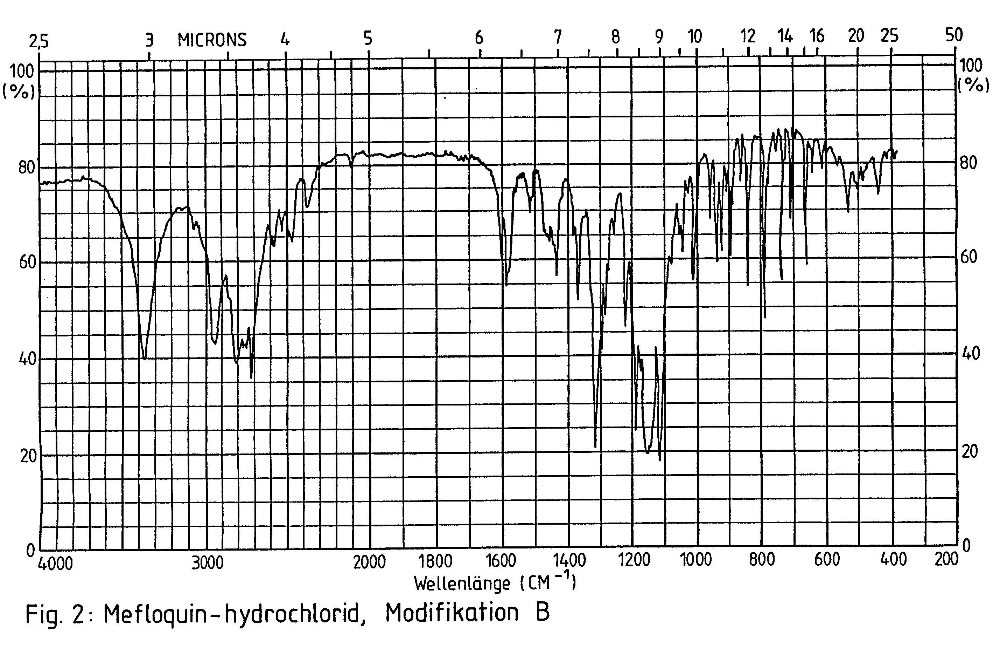

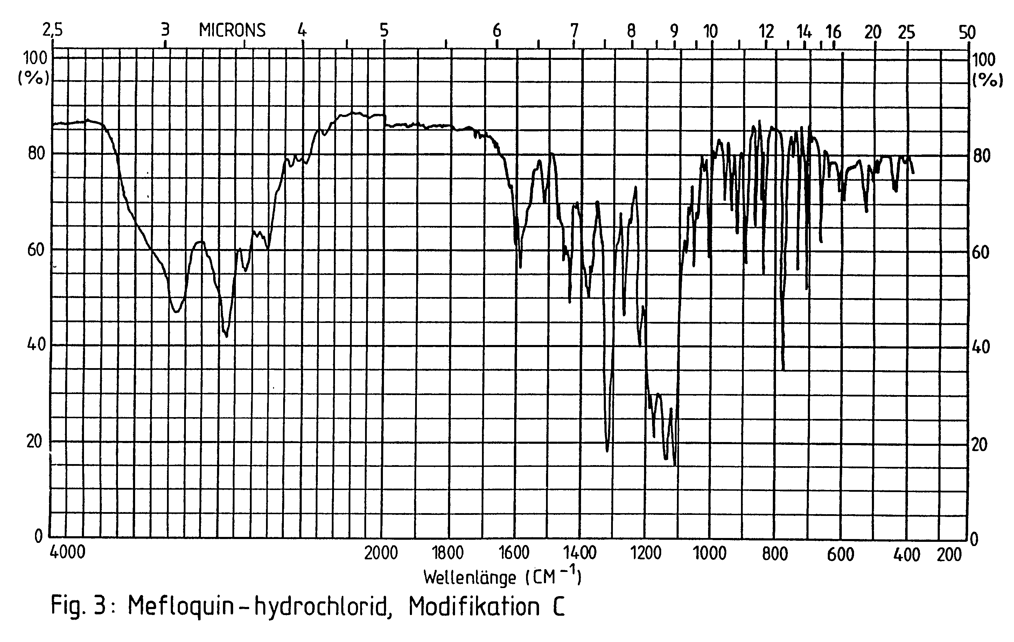

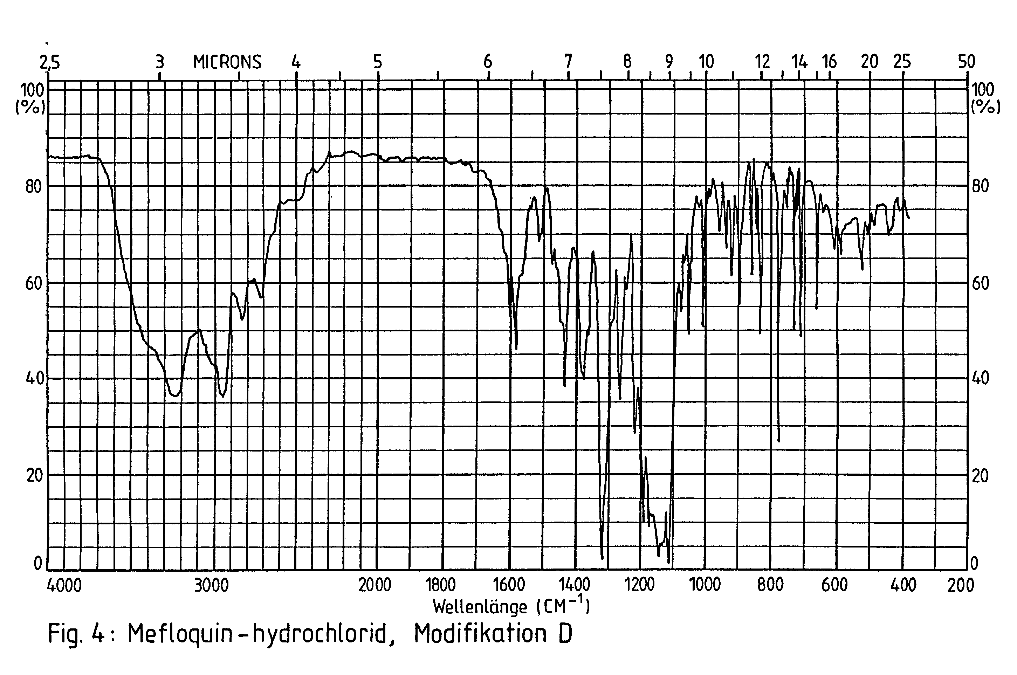

Hierbei wurde festgestellt, dass reines Mefloquinhdrochlorid in veschiedenen Modifikationen vorkommt, die sich kristallographisch oder aufgrund ihrer IR-Spektren voneinander unterscheiden lassen. So erhält man bei der Reinigung mittels Acetonitril eine Modifikation A, Mefloquin HCl, die durch das IR-Spektrum A (Figur 1) charakterisiert ist, während bei kurzzeitiger Behandlung mit Aceton die Modifikation B, Mefloquin HCl Aceton, und mit Wasser/Alkohol-Gemischen die Modifikation C, Mefloquin HCl 1/2 $H_2O$, erhalten wird (vgl. IR-Spektren B und C, Figuren 2 und 3). Bei längerer Behandlung von Mefloquin-hydrochlorid mit Alkohol/Wasser-Gemischen (ca. 50 bis 80 Vol%-Wasser) bildet sich die Modifikation D, Mefloquin HCl 1/2 $H_2O$, die die thermodynamisch stabilste ist (vgl. IR-Spektrum D, Figur 4).

Es wurde nun gefunden, dass durch Behandlung mit Methanol und/oder Aethanol enthaltend weniger als 30 Vol% Wasser Mefloquin-hydrochlorid eine polymorphe Modifikation E mit besonders interessanten Eigenschaften übergeführt wird.

So besitzen Tabletten, welche Mefloquin-hydrochlorid in der Modifikation E enthalten, eine bessere Löslichkeit und Bioverfügbarkeit als diejenigen, die Mefloquin-hydrochlorid in der Modifikation D enthalten, welches wie bereits erwähnt die thermodynamisch stabilste Modifikation ist.

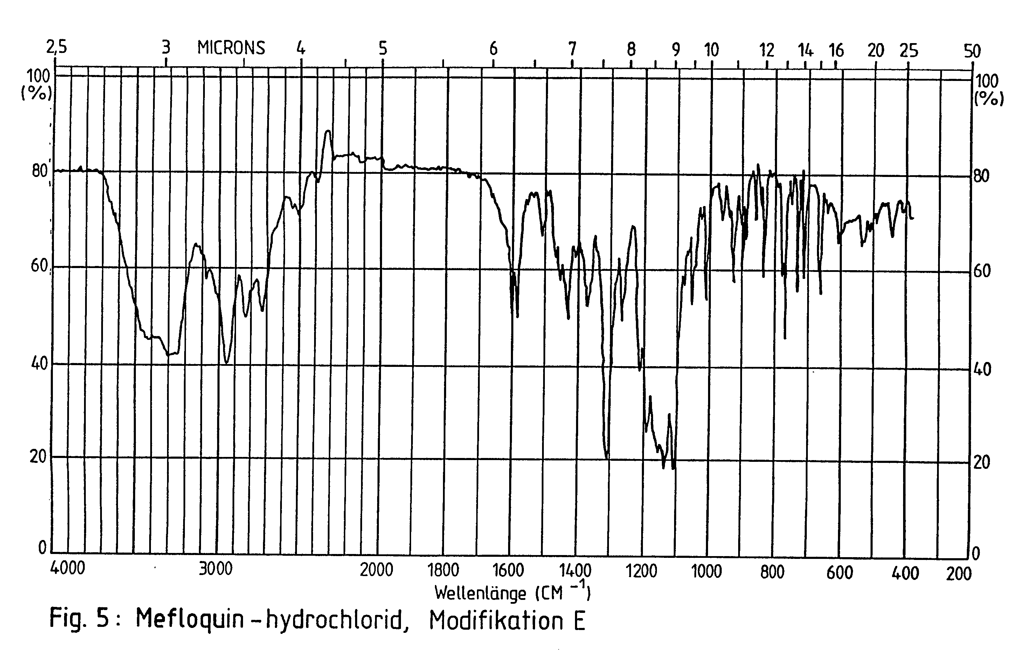

Das für die Modifikation E charakteristische IR-Spektrum wird in Figur 5 dargestellt. Ein Vergleich der IR-Spektren zeigt, dass die Unterschiede zwischen der Modifikation E und den Modifikationen A bis D hauptsächlich im Wellenzahl-Bereich 1100—1240 $cm^{-1}$ ersichtlich sind.

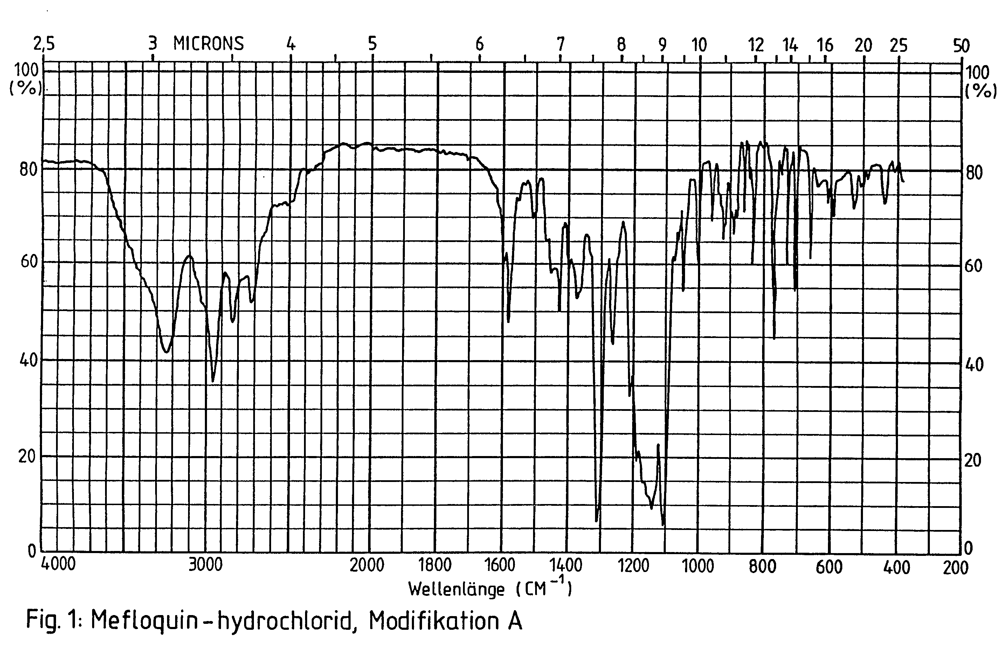

Die Modifikation E wird auch charakterisiert durch das Röntgenbeugungsdiagramm gemäss Figur 1 wobei sowohl der Ort, wie auch die relativen Intensitäten der Linien für das vorliegende Kristallgitter und dessen Zusammensetzung charakteristisch sind.

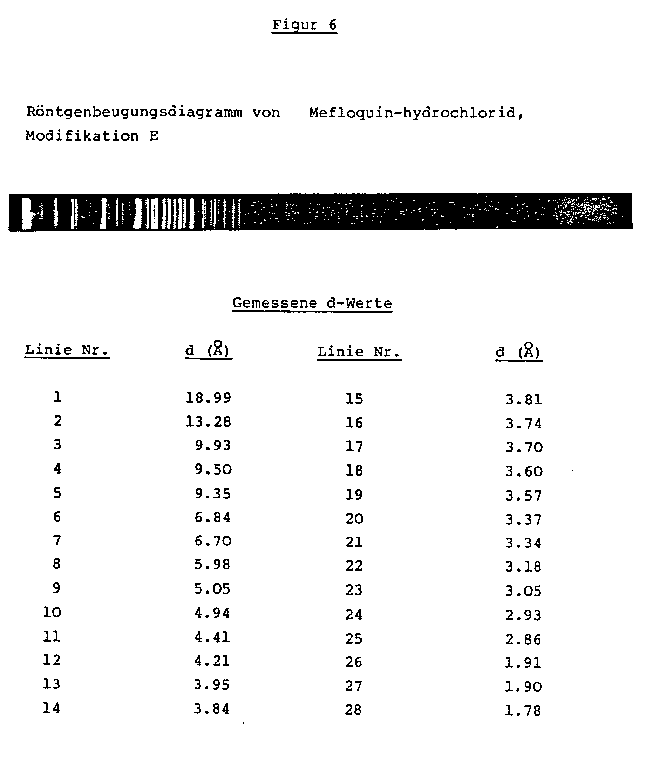

Schliesslich kann die Modifikation E auch anhand des Röntgenbeugungsdiagramms gemäss Figur 6 gemessener d-Werte charakterisiert werden. Der d-Wert lässt sich durch Umformung der Bragg'schen Gleichung darstellen:

$$d = \frac{\lambda}{2 \sin \theta}$$

λ=Wellenlänge der Röntgenstrahlung
θ=Beugungswinkel (auf der Abbildung in Figur 6 entsprechen 4 mm einem Beugungswinkel von 1°)
Die Ergebnisse werden in Figure 6 aufgeführt.

Die vorliegende Erfindung betrifft daher Mefloquinhydrochlorid in Form seiner Modifikation E charakterisiert durch das IR-Spektrum gemäss Figur 5 und/oder das Röntgenbeugungsdiagramm gemäss Figur 6, und/oder die aufgrund dieses Röntgenbeugungsdiagramms gemessenen d-Werte.

Weiter bezieht sich die vorliegende Erfindung auf ein Verfahren zur Herstellung von reinem Mefloquin-hydrochlorid in Form seiner Modifikation E, dadurch gekennzeichnet, dass man Mefloquin-hydrochlorid, das mindestens teilweise in einer anderen Modifikation vorliegt, gegebenenfalls in Gegenwart von weniger als 30 Vol% Wasser, mit Methanol und/oder Aethanol behandelt.

Die Herstellung der Modifikation E nach dem erfindungsgemässen Verfahren erfolgt vorzugsweise bei Zimmertemperatur wobei eine Behandlung während einigen Minuten in der Regel schon ausreicht. Sicherheitshalber wird jedoch vorgezogen, die Behandlung während wenigstens einer halben Stunde fortzusetzen.

Neben Methanol oder Aethanol bzw. einem Gemisch von Methanol und Aethanol können weniger als 30 Vol% Wasser, vorzugsweise weniger als 10 Vol% Wasser vorhanden sein. Besonders bevorzugt werden Methanol und Aethanol, ohne Zusatz von Wasser, in reiner Form verwendet.

Das als Ausgangsmaterial eingesetzte Mefloquin-hydrochlorid, welches mindestens teilweise in einer anderen Modifikation vorliegt als die Modifikation E, kann hergestellt werden durch Behandlung von erythro-/threo-α-2-Piperidyl-2,8-bis(trifluormethyl)-4-chinolin-methanol-Gemischen mit wässrigem Methanol oder Aethanol wobei die threo-Form in Lösung geht. Die Behandlung kann durch 30 minütiges bis mehrstündiges Rühren bei Zimmertemperatur oder unter Erwärmen bis auf etwa 80°C und anschliessende Abkühlung auf ca. 5°C (zur Erhöhung der Ausbeute) erfolgen. Der Wasseranteil am Lösungsmittelgemisch kann in weiten Grenzen variiert werden und liegt zweckmässigerweise bei 60—95 Vol-%, vorzugsweise zwischen 70 und 90 Vol-%. Bei der Aufarbeitung methanolischer Reaktionslösungen, wie sie beispielsweise bei der katalytischen Hydrierung von 2-Pyridyl-2,8-bis-(trifluormethyl)-4-chinolylketon in salzsaurem Methanol erhalten werden, wird zweckmässigerweise zunächst ein Teil des Methanols abgezogen und dann eine entsprechende Menge Wasser zugesetzt. Das so erhaltene Mefloquinhydrochlorid (Rohware) das zweckmässigerweise noch mit kaltem Wasser gewaschen und anschliessend getrocknet wird ist dann praktisch frei von der unerwünschten threo-Form und eignet sich als Ausgangsmaterial für das erfindungsgemässe Verfahren.

Diese Rohware ist chemisch zwar praktisch einheitlich, liegt im allgemeinen aber weder in einer einheitlichen Kristallform noch in der als Ausgangsmaterial bevorzugten thermodynamisch stabilsten Form (Modifikation D) vor. Die Modifikation D kann hergestellt werden indem man die Rohware längere Zeit, d.h. mindestens etwa 6 Stunden bis zu etwa 12 Stunden mit einem Alkohol, z.B. Methanol, Aethanol, Isopropanol, vorzugsweise aber Methanol, in Gegenwart von Wasser (ca. 50—80 Vol.-%) verrührt. Die Behandlung kann bei Zimmertemperatur oder unter Kühlung auf Temperaturen etwas über 0°C—zur Erhöhung der Ausbeute—erfolgen. Die Modifikationen A bis C können selbstverständlich ebenfalls als Ausgangsmaterial dienen.

Arzneimittel enthaltend Mefloquin-hydrochlorid in seiner Modifikation E sind ebenfalls Gegenstand der vorliegenden Erfindung. Sie können, neben pharmazeutisch akzeptablen Trägermaterialien, auch noch andere therapeutisch wertvolle Stoffe enthalten, wie z.B. Sulfadoxin und Pyrimethamin. So stellt eine Tablette enthaltend 250 mg Mefloquin-hydrochlorid in seiner Modifikation E, 500 mg Sulfadoxin und 25 mg Pyrimethamin ein besonders wertvolles Anti-malaria Mittel dar.

Die erfindungsgemässen Arzneimittel, welche vorzugsweise im Vergleich zu Mefloquin-hydrochlorid in Form seiner Modifikation E weniger als 10% Mefloquin-hydrochlorid in einer anderen Form enthalten, können als pharmazeutische Präparate mit direkter oder verzögerter Freigabe der Wirkstoffe in Mischung mit einem für die orale Applikation geeigneten organischen oder anorganischen inerten Trägermaterial, wie z.B. Wasser, Gelatine, Gummi arabicum, Milchzucker, Stärke, Magnesiumstearat, Talk, pflanzlichen Oelen, Polyalkylenglykolen, Vaseline, usw. verwendet werden. Die pharmazeutischen Präparate liegen vorzugsweise in fester Form, z.B. als Tabletten, Dragées, Kapseln, vor. Gegebenenfalls sind sie sterilisiert und bzw. oder enthalten weitere Hilfsstoffe, wie Konservierungs-, Stabilisierungs-, Netz- oder Emulgiermittel, Mittel zur geschmacklichen Verbesserung, Salze zur Veränderung des osmotischen Druckes oder Puffersubstanzen. Die Herstellung der pharmazeutischen Präparate kann in der jedem Fachmann geläufigen Weise erfolgen.

Eine bevorzugte Dosiseinheit für Mefloquin-hydrochlorid in Form der Modifikation E liegt zwischen 1 und 15 mg/kg.

Die erfindungsgemässen pharmazeutischen Präparate eignen sich zur kurativen Behandlung der Malaria (alle Formen) mit einer Einzeldosis in Form von beispielsweise einer oder mehreren Tabletten enthaltend vorzugsweise 250 mg Mefloquin, je nach Körpergewichts des Patienten und Anweisung des Arztes, als auch zur Prophylaxe und Suppressivtherapie in Intervallen von 1—4 Wochen.

Beispiel 1

1,5 g Mefloquin-hydrochlorid (Modifikation D) wurden in 5 ml reinem Aethanol suspendiert und während 30 Minuten in einem Vibromischer bei 25°C aequilibriert. Nach Zentrifugieren und Abpipettieren der überstehenden Lösung wurde im Vakuumtrockner während zwei Stunden bei Raumtemperatur getrocknet. Man erhielt Mefloquin-hydrochlorid in Form seiner Modifikation E.

Beispiel 2

1,5 g Mefloquin-hydrochlorid (Modifikation D) wurden in 5 ml reinem Methanol suspendiert und während 30 Minuten in einem Vibromischer bei 25°C aequilibriert. Nach Zentrifugieren und Abpipettieren der überstehenden Lösung wurde im Vakuumtrockner während zwei Stunden bei Raumtemperatur getrocknet. Man erhielt Mefloquin-hydrochlorid in Form seiner Modifikation E.

Beispiel 3

1,5 g Mefloquin-hydrochlorid (Modifikation D) wurden in 5 ml verschiedenen Aethanol/Wasser-Gemischen suspendiert und während 30 Minuten, 24 Stunden oder 72 Stunden in einem Vibromischer bei 25°C aquilibriert. Nach Zentrifugieren und Abpipettieren der überstehenden Lösung wurde im Vakuumtrockner während zwei Stunden bei Raumtemperatur getrocknet. Wie aus der nachstehenden Tabelle 1 hervorgeht erhielt man Mefloquin-hydrochlorid in Form seiner Modifikation E, wobei die Aequilibrierungsdauer vom Wasseranteil im Lösungsmittelgemisch abhängt.

# EP 0 137 375 B1

TABELLE 1

| Lösungsmittel | Kristallographische Form von Mefloquin-hydrochlorid | | |
|---|---|---|---|
| Aethanol:Wasser | nach 30 Min. | nach 24 Std. | nach 72 Std. |
| 70:30 | Mod D | Mod. E | Mod. E |
| 90:10 | Mod. D+E | Mod. E | Mod. E |
| 92:8 | Mod. D+E | Mod. E | Mod. E |
| 95:5 | Mod. E | Mod. E | Mod. E |

Beispiel 4

1,5 g Mefloquin-hydrochlorid (Modifikation A) wurden in 5 ml reinem Aethanol suspendiert und während 30 Minuten in einem Vibromischer bei 25°C aequilibriert. Nach Zentrifugieren und Abpipettieren der überstehenden Lösung wurde im Vakuumtrockner während zwei Stunden bei Raumtemperatur getrocknet. Man erhielt Mefloquin-hydrochlorid in Form seiner Modifikation E.

Beispiel 5

1,5 g Mefloquin-hydrochlorid (Modifikation C) wurden in 5 ml reinem Aethanol suspendiert und während 30 Minuten in einem Vibromischer bei 25°C aequilibriert. Nach Zentrifugieren und Abpipettieren der überstehenden Lösung wurde im Vakuumtrockner während zwei Stunden bei Raumtemperature getrocknet. Man erhielt Mefloquin-hydrochlorid in Form seiner Modifikation E.

Beispiel 6

Tablette enthaltend:

| | |
|---|---|
| Mefloquin-hydrochlorid Modifikation (E) | 274,12 mg (entsprechend 250 mg Mefloquin |
| Poloxalkol | 2,94 mg |
| Mikrokristalline Cellulose | 63,02 mg |
| Crosspovidone | 40,0 mg |
| Ammonium-Calcium-Alginat | 11,76 mg |
| Maisstärke | 28,88 mg |
| Lactose | 50,58 mg |
| Talk | 11,0 mg |
| Magnesiumstearat | 11,0 mg |
| | 493,3 mg |

Herstellung

Mefloquin-hydrochlorid (Modifikation D) wird mit einer Lösung von Poloxalkol in Alkohol befeuchtet und geknetet; sodann wird getrocknet und gesiebt. Man erhält ein Granulat, enthaltend Mefloquin-hydrochlorid in der Modifikation E.

Das Granulat wird mit Mikrokristalliner Cellulose, Lactose, Maisstärke, Crosspovidone (Teil) sowie Ammonium-Calcium-Alginat gemischt. Die Pulvermischung wird mit einem Wasser/Alkohol-Gemisch befeuchtet und geknetet. Sodann wird granuliert, getrocknet und gesiebt.

Dem erhaltenen Granulat werden Crosspovidone (2. Teil) sowie eine gesiebte Mischung aus dem Talk und dem Magnesiumstearat zugefügt: sämtliche Pulverbestandteile werden miteinander vermischt und das Pressgut wird zu Tabletten entsprechender Grösse verpresst.

4

Beispiel 7

Tablette enthaltend:

| | |
|---|---|
| Mefloquin-hydrochlorid, Modifikation (E) | 274,12 mg (entsprechend 250 mg Mefloquin) |
| Sulfadoxin | 500,0 mg |
| Pyrimethamin | 25,0 mg |
| Dioctylnatriumsulfosuccinat | 1,0 mg |
| Methylcellulose | 7,5 mg |
| Natriumcarboxymethylstärke | 35,0 mg |
| Mikrokristalline Cellulose | 48,38 mg |
| Magnesiumstearat | 9,0 mg |
| | 900,0 mg |

Herstellung

Mefloquin-hydrochlorid (Modifikation D) wird mit Alkohol befeuchtet und geknetet; sodann wird getrocknet und gesiebt. Man erhält ein Granulat, enthaltend Mefloquin-hydrochlorid in der Modifikation E.

In eine wässrige Lösung von Dioctylnatriumsulfosuccinat und Methylcellulose werden Sulfadoxin, Pyrimethamin, Natriumcarboxymethylstärke und das Granulat von Mefloquinhydrochlorid eingearbeitet und geknetet. Sodann wird granuliert, getrocknet und gesiebt.

Dem Granulat werden gesiebte Mikrokristalline Cellulose und gesiebtes Magnesiumstearat zugesetzt; sämtliche Pulverbestandteile werden miteinander gemischt und das Pressgut wird zu Tabletten verpresst.

## Patentansprüche

1. Mefloquin-hydrochlorid in Form einer Modifikation E, erhältlich indem man Mefloquin-hydrochlorid, das mindestens teilweise in anderer Modifikation vorliegt, gegebenenfalls in Gegenwart von weniger als 30 Vol.-% Wasser, mit Methanol und/oder Aethanol behandelt.

2. Mefloquin-hydrochlorid in Form seiner Modifikation E charakterisiert durch das IR-Spektrum gemäss Figur 5 und/oder das Röntgenbeugungsdiagramm und/oder die d-Werte gemäss Figur 6.

3. Mefloquin-hydrochlorid in Form seiner Modifikation E als pharmazeutischer Wirkstoff.

4. Mefloquin-hydrochlorid in Form seiner Modifikation E als Anti-malaria Mittel.

5. Verfahren zur Gewinnung von reinem Mefloquin-hydrochlorid in Form seiner Modifikation E, dadurch gekennzeichnet, dass man Mefloquin-hydrochlorid, das mindestens teilweise in anderer Modifikation vorliegt, gegebenenfalls in Gegenwart von weniger als 30 Vol.-% Wasser, mit Methanol und/ oder Aethanol behandelt.

6. Verfahren nach Anspruch 5, dadurch gekennzeichnet, dass gegebenenfalls weniger als 10 Vol.-% Wasser vorhanden ist.

7. Verfahren nach Anspruch 5 oder 6, dadurch gekennzeichnet, dass reines Methanol eingesetzt wird.

8. Verfahren nach Anspruch 5 oder 6, dadurch gekennzeichnet, dass reines Aethanol eingesetzt wird.

9. Verfahren nach einem der Ansprüche 5 bis 8, dadurch gekennzeichnet, dass als Ausgangsmaterial die thermodynamisch stabilste Form D von Mefloquin-hydrochlorid verwendet wird.

10. Pharmazeutisches Präparat enthaltend Mefloquinhydrochlorid in Form seiner Modifikation E.

11. Pharmazeutisches Präparat gemäss Anspruch 10, dadurch gekennzeichnet, dass es neben Mefloquin-hydrochlorid in Form seiner Modifikation E weitere therapeutische aktive Wirksubstanzen enthält.

12. Pharmazeutisches Präparat gemäss Ansprüche 10 und 11, dadurch gekennzeichnet, dass es neben Mefloquinhydrochlorid in Form seiner Modifikation E Pyrimethamin und Sulfodoxin enthält.

13. Pharmazeutisches Präparat gemäss einem der Ansprüche 10 bis 12, dadurch gekennzeichnet, dass es im Vergleich zu Mefloquin-hydrochlorid in Form seiner Modifikation E weniger als 10% Mefloquin-hydrochlorid in Form einer anderen Modifikation enthält.

## Revendications

1. Chlorhydrate de méfloquine sous sa forme E, obtenu en traitant un chlorhydrate deméfloquine, en partie au moins sous une autre forme physique, éventuellement en présence de moins de 30% en volume d'eau, par le méthanol et/ou l'éthanol.

2. Chlorhydrate de méfloquine sous sa forme E, caractérisé par le spectre IR de la figure 5 et/ou le diagramme de diffraction de rayons X et/ou les valeurs de d de la figure 6.

3. Chlorhydrate de méfloquine, sous sa forme E, en tant que substance active pharmaceutique.

4. Chlorhydrate de méfloquine sous la forme E, en tant qu'agent antipaludéen.

5. Procédé pour obtenir du chlorhydrate de méfloquine pur sour sa forme E, caractérisé en ce que l'on traite un chorhydrate de méfloquine, en partie au moins sous une autre forme physique, éventuellement en présence de moins de 30% en volume d'eau, par du méthanol et/ou de l'éthanol.

6. Procédé selon la revendication 5, caractérisé en ce que l'on opère le cas échéant en présence de moins de 10% en volume d'eau.

7. Procédé selon la revendication 5 ou 6, caractérisé en ce que l'on utilise du méthanol pur.

8. Procédé selon la revendication 5 ou 6, caractérisé en ce que l'on utilise de l'éthanol pur.

9. Procédé selon l'une des revendications 5 à 8, caractérisé en ce que l'on utilise en tant que produit de départ la forme D, possédant la plus forte stabilité thermodynamique, du chlorhydrate de mefloquine.

10. Composition pharmaceutique contenant du chlorhydrate de méfloquine sous sa forme E.

11. Composition pharmaceutique selon la revendication 10, caractérisée en ce qu'elle contient, en plus du chlorhydrate de méfloquine sous sa forme E, d'autres substances actives thérapeutiques.

12. Composition pharmaceutique selon les revendications 10 et 11, caractérisée en ce qu'elle contient, en plus du chlorhydrate de méfloquine sous sa forme E, du Pyrimethamine et du Sulfadoxine.

13. Composition pharmaceutique selon l'une des revendications 10 à 12, caractérisée en ce qu'elle contient, avec le chlorhydrate de méfloquine sous sa forme E, moins de 10% de chlorhydrate de méfloquine sous une autre forme physique.

## Claims

1. Mefloquine hydrochloride in the form of its modification E, obtainable by treating mefloquine hydrochloride, which is present at least partially in another modification, with methanol and/or ethanol, optionally in the presence of less than 30 vol.% water.

2. Mefloquine hydrochloride in the form of its modification E characterized by the IR spectrum in accordance with Figure 5 and/or the X-ray diffraction pattern and/or the d-values in accordance with Figure 6.

3. Mefloquine hydrochloride in the form of its modification E as a pharmaceutically active substance.

4. Mefloquine hydrochloride in the form of its modification E as an anti-malarial agent.

5. A process for the manufacture of pure mefloquine hydrochloride in the form of its modification E, characterized by treating mefloquine hydrochloride, which is present at least partially in another modification, with methanol and/or ethanol, optionally in the presence of less than 30 vol.% water.

6. A process according to Claim 5, characterized in that less than 10 vol.% water is optionally present.

7. A process according to Claim 5, or 6, characterized in that pure methanol is used.

8. A process according to Claim 5 or 6, characterized in that pure ethanol is used.

9. A process according to any one of Claims 5 to 8, characterized in that the thermodynamically most stable form D of mefloquine hydrochloride is used as the starting material.

10. A pharmaceutical preparation containing mefloquine hydrochloride in the form of its modification E.

11. A pharmaceutical preparation in accordance with Claim 10, characterized in that it contains further therapeutically active substances in addition to mefloquine hydrochloride in the form of its modification E.

12. A pharmaceutical preparation in accordance with Claim 10 and 11, characterized in that it contains pyrimethamine and sulfoxidine in addition to mefloquine hydrochloride in the form of its modification E.

13. A pharmaceutical preparation in accordance with any one of Claims 10 to 12, characterized in that it contains in comparison to mefloquine hydrochloride in the form of its modification E less than 10% of mefloquine hydrochloride in the form of another modification.

Fig. 1: Mefloquin-hydrochlorid, Modifikation A

EP 0 137 375 B1

Fig. 2: Mefloquin-hydrochlorid, Modifikation B

EP 0 137 375 B1

Fig. 3: Mefloquin-hydrochlorid, Modifikation C

Wellenlänge (CM$^{-1}$)

MICRONS

(%)

EP 0 137 375 B1

Fig. 4: Mefloquin-hydrochlorid, Modifikation D

4

Wellenlänge (CM $^{-1}$)

Fig. 5 : Mefloquin – hydrochlorid, Modifikation E

EP 0 137 375 B1

## Figur 6

Röntgenbeugungsdiagramm von    Mefloquin-hydrochlorid,
Modifikation E

### Gemessene d-Werte

| Linie Nr. | d (Å) | Linie Nr. | d (Å) |
|-----------|-------|-----------|-------|
| 1 | 18.99 | 15 | 3.81 |
| 2 | 13.28 | 16 | 3.74 |
| 3 | 9.93 | 17 | 3.70 |
| 4 | 9.50 | 18 | 3.60 |
| 5 | 9.35 | 19 | 3.57 |
| 6 | 6.84 | 20 | 3.37 |
| 7 | 6.70 | 21 | 3.34 |
| 8 | 5.98 | 22 | 3.18 |
| 9 | 5.05 | 23 | 3.05 |
| 10 | 4.94 | 24 | 2.93 |
| 11 | 4.41 | 25 | 2.86 |
| 12 | 4.21 | 26 | 1.91 |
| 13 | 3.95 | 27 | 1.90 |
| 14 | 3.84 | 28 | 1.78 |